# EUROPEAN PATENT SPECIFICATION

(11) **EP 0 502 024 B1**
(45) Date of publication and mention of the grant of the patent: **10.07.1996**
(21) Application number: 90917144.9
(22) Date of filing: 22.11.1990
(51) Int. Cl.: C07D 401/04, C07D 405/04, C07D 409/04

(54) **4-HETEROARYL PIPERIDINE INTERMEDIATES AND THEIR PREPARATION**
4-HETEROARYL-PIPERIDIN-ZWISCHENPRODUKTE UND DEREN HERSTELLUNG
INTERMEDIAIRES DE 4-HETEROARYLE PIPERIDINE ET LEUR PREPARATION

(30) Priority: 22.11.1989 DK 5882/89
(43) Date of publication of application: 09.09.1992
(73) Proprietor: NOVO NORDISK A/S, DK-2880 Bagsvaerd (DK)
(72) Inventor: JAKOBSEN, Palle, DK-3500 Vaerlose (DK); SONNEWALD, Ursula, N-7048 Trondheim (NO); TREPPENDAHL, Svend, DK-2830 Virum (DK)
(86) International application number: DK9000304
(87) International publication number: WO9107398

(56) References cited:
- EP-A- 0 190 496
- EP-A- 0 223 334
- US-A- 4 007 196

## Description

This invention relates to new heteroaryl piperidine carbinols which are suitable as intermediates for the preparation of the compounds disclosed in EP 505 465 and to a novel chemical process for preparing said carbinols.

EP 505 465 discloses piperidine compounds having activity against calcium overload in brain cells, and being useful in the treatment of anoxia, traumatic injury, ischemia, migraine and epilepsy.

This invention relates to new compounds of formula I 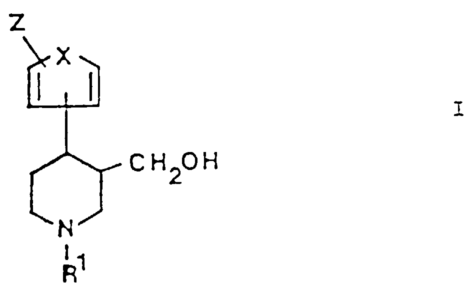 in which X is O, S, or NR where R is H or C₁₋₄-alkyl; Z is hydrogen or straight or branched C₁₋₈-alkyl; R¹ is H or straight or branched C₁₋₈-alkyl.

The invention also relates to methods of preparing a compound of formula I.

These methods which use easily accessible commercially available starting materials comprise:
a) reacting a compound of formula IV

   R³OCCH₂COOR² IV

   with a compound of the formula V 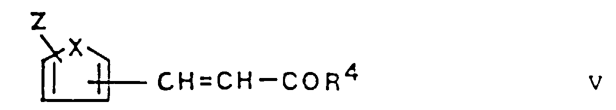 under basic conditions eg. using alkoxide in ethanol, wherein X, Z, R, R¹ and R² have the meanings defined above and R³ being C₁₋₂-alkoxy when R⁴ is NHR¹ or R³ being NHR¹ when R⁴ is C₁₋₂-alkoxy, for the preparation of a compound of formula II 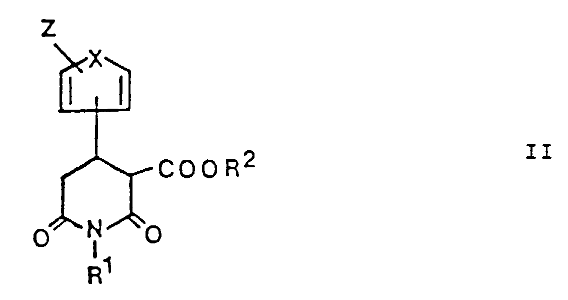 wherein X, Z, R and R¹ have the meanings defined above and R² is C₁₋₂-alkyl, and
b) reduction of the compound of formula II wherein X, Z, R, R¹ and R² have the meanings defined above, with metal hydride eg. LiAlH₄ or AlH₃ in ether or THF giving a compound of formula I wherein X, Z, R and R¹ have the meanings defined above,
c) reduction of a compound of formula III 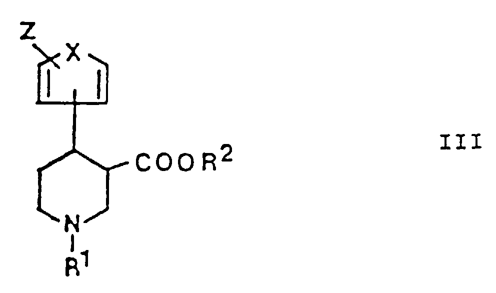 wherein X, Z, R, R¹ and R² have the meanings defined above with a metal hydride eg. LiAlH₄ or AlH₃ giving a compound of formula I wherein X, Z, R and R¹ have the meanings defined above;
   compounds of formula III can be prepared conveniently from Arecoline-type derivatives and metal organic derivatives of heteroaromates using well known procedures,
d) compounds of formula I may also be prepared by reacting compound of formula I wherein Z is H, with reagents causing heteroaromatic substitution using well known procedures,
e) compounds of formula I may be prepared by metal hydride reduction of a compound of formula VI 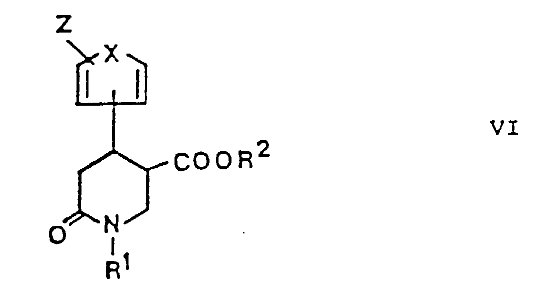 wherein X, Z, R, R¹ and R² have the meanings defined above,
f) compounds of formula II may be prepared in a one pot reaction using a compound of formula VII wherein X, Z and R have the meanings defined above 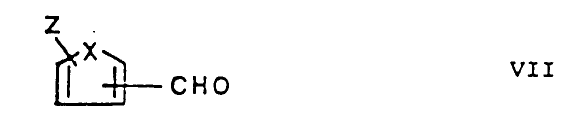 as starting material. The reaction is carried out using ethyl acetate as solvent and methoxide or ethoxide as base. After initial reaction between VII and solvent a compound of formula VIII wherein R¹ and R² have the meanings defined above,

   R²OOCCH₂CONHR¹ VIII

   is added resulting in the formation of compounds of formula II wherein X, Z, R, R¹ and R² have the meanings defined above.

The invention also relates to the compounds of formula (II), the specific compounds (5), (1), (21), (8) , (2) , (6) , (11), (13), (20), (17) and (15) described below and the method of preparing compound (1) as described in Example 1.

### EXAMPLE 1

3-hydroxymethyl-1-methyl-4-(2-thienyl)-piperidine (1)
3-methoxycarbonyl-1-methyl-4-(2-thienyl)-piperidine (2) was prepared from Arecoline, HBr (52 g), 2-bromothiophene (41 ml) and Mg-turnings (9.9 g) as described by Plati et. al. (J. Org. Chem. 22 (1957) 261). The resulting product was purified by distillation giving 15 g cis/trans mixture b.p. 50-120°C / 147 Pa (1.1 mm Hg).

30 g of this product was reduced with LiAlH₄ (5 g) in dry ether (200 ml) by reflux for 30 min. in N₂-atmosphere. The well known rinse up procedure gave a hard oil (21 g) identified as 3-hydroxymethyl-1-methyl-4(2-thienyl)-piperidine(1) by ¹H NMR (CDCl₃ ), : 6.7 - 7.3 (3H,m); 3.8- 4.5 (1H, broad) 3.5-3.7 (2H,m); 2.8-3.5 (3H,m); 2.2 (3H,s); 1.8-2.8 (5H,m)

### EXAMPLE 2

3-(2-thienyl)-propenoyl chloride (3) was prepared by dropwise addition of thionyl chloride (50 ml) to 3-(2-thienyl)-propenoic acid (25 g) and subsequent heating to 60°C for 2 hours. Excess of SOCl₂ was evaporated in vacuo, CH₂Cl₂ was added and the reaction mixture was evaporated to dryness yielding 28 g of (3).

N-pentyl-3-(2-thienyl)-propenoic amide (4) was prepared from (3) (28 g) dissolved in dry toluene (200 ml) pentyl amine (25 ml) and triethyl amine (70 ml) was added under cooling (ice bath). Stirring for 1 hour. The precipitate of triethylammonium chloride was removed by filtration, the filtrate was evaporated to dryness and the resulting mass treated with ether giving (4) as colourless crystals (22.8 g). ¹H NMR (CDCl₃), : 0.7-1.1 (3H,m); 1.1-1.7(6H,m); 3.0-3.4 (2H, dist.q ), 6.2-8.2 (6H,m).

3-ethoxycarbonyl-1-pentyl-4-(2-thienyl)-piperidine 2,6-dione (5) Sodium (3 g) was dissolved in abs. ethanol (60 ml), diethyl malonate (20 ml) dissolved in abs. ethanol (30 ml) was added followed by a slurry of (4) (22.8 g) in abs. ethanol (50 ml). Reflux for 4 hours, the ethanol was evaporated, toluene added (100 ml), and the reflux continued overnight. After cooling the formed precipitate was isolated, dissolved in 1M HCl and extracted several times with ether. The combined ether phases were evaporated giving an oil which was purified on silica gel using CH₂Cl₂/CH₃OH 9/l as eluent. Yield 22.6 g of (5) as an oil identified by ¹H NMR (CDCl₃), : 0.7-0.95 (3H,m); 1.0-1.5 (9H,m); 2.9-3.2 (2H,m) 4.15 (2H,q); 3.6-4.15 (4H,m); 6.8-7.2 (3H,m).

3-hydroxymethyl-1-pentyl-4-(2-thienyl)-piperidine (6) was prepared from (5) (22.6 g) by reduction with LiAlH₄ (13 g) in dry THF. Reflux for 3 hours under N₂. Using the well known work up procedure gave a hard oil (4 g) identified by ¹H NMR (CDCl₃), : 0.7-1.1 (3H,m); 1.1-1.7 (6H,m); 1.7-2.1 (4H,m); 2.1-3.9 (9H,m); 6.8-7.2 (3H,m).

### EXAMPLE 3

N-pentyl-3-(3-thienyl)-propenoic amide (7) was prepared as described for (4). ¹H NMR (CDCl₃), : 0.7-1,1 (3H,m); 1.1-1.7 (6H,m); 3.2-3.6 (2H, dist. q); 6.1-7.8 (6H,m).

3-ethoxycarbonyl-1-pentyl-4-(3-thienyl)-piperidine-2,6-dione (8) was prepared as described for (5). 32 g (7) gave 41 g of crude (8) which was used without further purification. Reduction and work up as described for the preparation of (6) gave 7.7 g of crystalline 3-hydroxymethyl-1-pentyl-4-(3-thienyl)-piperidine (9). M.p. 96.5-97.5°C.

### EXAMPLE 4

### (+)-1-butyl-3-ethoxycarbonyl-4-(2-thienyl)-2,6-piperidinedione (10)

A solution of 2-thiophenecarbaldehyde (22.4 g) in ethyl acetate (20 ml) was added to a slurry of sodium ethanolate (32.6 g) in ethyl acetate (200 ml). The temperature was kept at 10°C and the mixture stirred for one hour. A solution of ethyl N-butylamidomalonate (41.2 g) in ethyl acetate (40 ml) was slowly added to the mixture whilst keeping the temperature below 5°C. The mixture was stirred for 18 hours at 20°C and neutralized with 25% acetic acid (130 ml). The water phase was discharged and the organic phase extracted twice with saturated sodium chloride solution (2x50 ml). The organic phase was evaporated, the residue dissolved in toluene (200 ml), dried with potassium carbonate and evaporated to give the crude product. Yield 72 g of an oil identified by ¹H NMR (CDCl₃), : 0.7-1.4 (10H,m); 2.7-4.2 (8H,m); 6.7-7.3 (3H,m).

### (±)-1-butyl-3-hydroxymethyl-4-(2-thienyl)-piperidine (11)

A solution of crude (±)-1-butyl-3-ethoxycarbonyl-4-(2-thienyl)-2,6-piperidindione (72 g) in toluene (100 ml) was added to a slurry of LiAlH₄ (15.2 g) in THF (100 ml) and toluene (50 ml). The temperature was kept below 10°C during the addition. The reaction mixture was stirred for 18 hours and decomposed by careful addition of water (75 ml) keeping the temperature below 10°C. The hydrolyzed mixture was stirred for 1 hour before the precipitated salts was filtered off. The filtrate was evaporated to give an oil (33 g) which was recrystallized from ethyl acetate (50 ml), filtered off and dried to give the title compound (17 g), m.p. 89.7-90.1°C.

### EXAMPLE 5

(+-)-1-butyl-3-ethoxycarbonyl-4-(5-methyl-2-furanyl)-2,6-piperidinedione (12)
was prepared from 5-methyl- 2-furancarbaldehyde (22 g) as described for compound (10). Yield 59.5 g of an oil (80% pure by HPLC). Identified by ¹H NMR (CDCl₃) : 0.7-1.6 (10H, m), 2.2 - 2.4 (3H, d); 2.8 - 4.5 (8H, m); 5.8-7.5 (2H, m).

(+-)-1-butyl-3-hydroxymethyl-4-(5-methyl-2-furanyl)-piperidine (13) was prepared from crude (12) (59 g) by reduction with LiAlH₄, as described for (11). Yield 22 g of (13). M.p. 83.5°C.

### EXAMPLE 6

### (+-)-1-butyl-3-ethoxycarbonyl-4-(1-methyl-2-pyrrolyl)-2,6-piperidinedione (14)

was prepared from 1-methyl-2-pyrrolecarbaldehyde (10.2 g) and ethyl N-butylamidomalonate (15.4 g) in ethyl acetate as described for compound (10). The crude product (27 g) was subsequently reduced without further purification as described for compound (11). Yield 14 g of

(+-)-1-butyl-3-hydroxymethyl-4-(1-methyl-2-pyrrolyl)-piperidine (15) precipitated as the oxalate. ¹H NMR (CDCl₃) : 0.7-1.1 (3H, m); 1.1 - 2.2 (6H, m); 2.5 - 3.8 (10H, m), 3.5 (3H, s), 5.7 - 6.0 (2 H, m); 6.1 - 6.7 (1H, m).

### EXAMPLE 7

### 3-Ethoxycarbonyl-1-(2-methylbutyl)-4-(3-thienyl)-2,6-piperidinedione (16)

was prepared from 3-thiophenecarbaldehyde (20 g) and ethyl N-(2-methylbutylamidomalonate) (35 g) in ethyl acetate as described for compound (10). The crude product (60 g oil) was reduced in THF by means of LiAlH₄ as described for compound (11) giving

### 3-hydroxymethyl-1-(2-methylbutyl)-4-(3-thienyl)-piperidine (17)

The crude product (23 g) was purified on silica gel using ethyl acetate as eluent.

Mass spectrum (M⁺: 267) degradation in accordance with proposed structure. m.p. 88.8-90.2°C.

### EXAMPLE 8

The following compounds were prepared exactly as described for (17) using the appropriate substituted thiophenecarbaldehyde and ethyl amidomalonate. The dione intermediate was used without purification.

3-hydroxymethyl-1-pentyl-4-(2-thienyl)piperidine (18)
Yield 17.6%; m.p. 107.6°C.

1-butyl-3-hydroxymethyl-4-(2-thienyl)piperidine (19)
Yield 27.2%; m.p. 90.9°C.

1-butyl-3-hydroxymethyl-4-(3-thienyl)piperidine (20)
Yield 27.9%; m.p. 81.7°C.

3-hydroxymethyl-1-pentyl-4-(3-thienyl)piperidine (21)
Yield 27.5%; m.p. 93.9°C.

## Claims

1. A compound of formula I 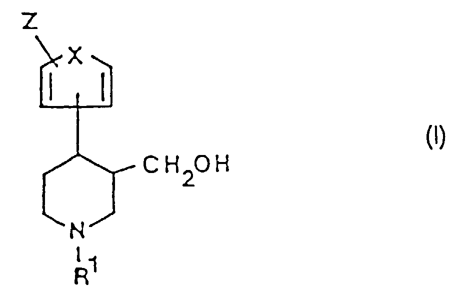 wherein X is O, S or NR wherein R is hydrogen or C₁₋₄-alkyl; and Z is hydrogen or straight or branched C₁₋₈-alkyl; and R¹ is hydrogen or straight or branched C₁₋₈-alkyl.

2. A compound of formula II 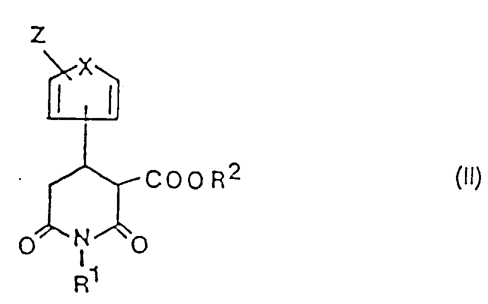 wherein X is O, S or NR wherein R is hydrogen or C₁₋₄-alkyl; and Z is hydrogen or straight or branched C₁₋₈-alkyl; and R¹ is hydrogen or straight or branched C₁₋₈-alkyl; and R² is C₁₋₂-alkyl.

3. A process for the preparation of a compound according to claim 1, comprising reacting a compound of formula IV
R³OCCH₂COOR² (IV)
with a compound of formula V 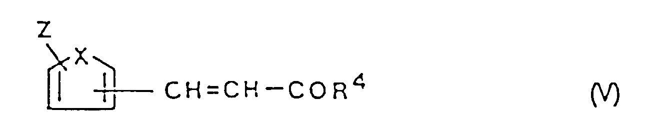 under basic conditions, wherein X, Z, R and R² have the meanings defined above and R³ being C₁₋₂-alkoxy when R⁴ is NHR¹ or R³ being NHR¹ when R⁴ is C₁₋₂-alkoxy wherein R¹ has the meaning defined above, to form a compound of formula II 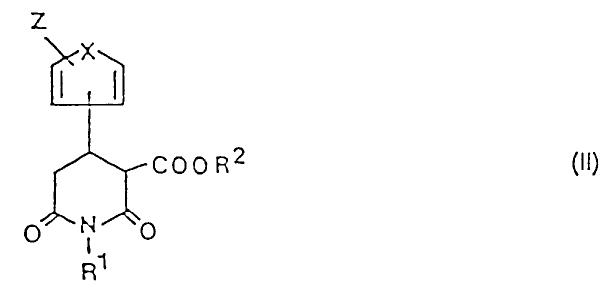 wherein X, Z, R, R¹ and R² have the meanings defined above, and reduction of a compound of formula II with a metal hydride e.g. LiAlH₄ to form a compound of formula I.

4. A process for the preparation of a compound according to claim 1, comprising reacting a compound of formula VII 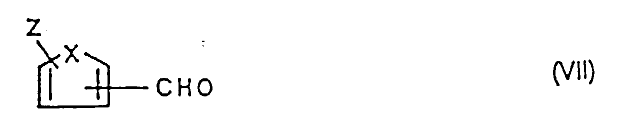 with a compound of formula VIII
R²OOCCH₂CONHR¹ (VIII)
in ethyl acetate using ethoxide or methoxide as base, wherein X, Z, R, R¹ and R² have the meanings defined above, to form a compound of formula II 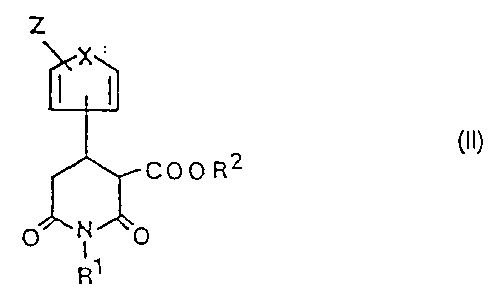 wherein X, Z, R, R¹ and R² have the meanings defined in claim 2 and reduction of a compound of formula II with a metal hydride e.g. LiAlH₄ to form a compound of formula I.

5. A process for the preparation of 3-hydroxymethyl-1-methyl-4-(2-thienyl)-piperidine, comprising reacting arecoline and HBr with 2-bromothiophene in the presence of Mg turnings, to form the compound 3-methoxycarbonyl-1-methyl-4-(2-thienyl)-piperidine, and reduction of said compound with LiAlH₄.

6. A compound of formula II which is 3-ethoxycarbonyl-1-pentyl-4-(2-thienyl)-piperidine-2,6-dione.

7. A compound of formula I which is 3-hydroxymethyl-1-methyl-4-(2-thienyl)-piperidine.

8. A compound of formula I which is 3-hydroxymethyl-1-pentyl-4-(3-thienyl)-piperidine.

9. A compound of formula II which is 3-ethoxycarbonyl-1-pentyl-4-(3-thienyl)-piperidine-2,6-dione.

10. A compound of formula I which is 3-hydroxymethyl-1-pentyl-4-(2-thienyl)-piperidine.

11. A compound of formula I which is 1-butyl-3-hydroxymethyl-4-(2-thienyl)-piperidine.

12. A compound of formula I which is 1-butyl-3-hydroxymethyl-4-(5-methyl-2-furanyl)-piperidine.

13. A compound of formula I which is 1-butyl-3-hydroxymethyl-4-(3-thienyl)-piperidine.

14. A compound of formula I which is 3-hydroxymethyl-1-(2-methyl-butyl)-4-(3-thienyl)-piperidine.

15. A compound of formula I which is 1-butyl-3-hydroxymethyl-4-(1-methyl-2-pyrrolyl)-piperidine.

## Patentansprüche

1. Verbindung der Formel I 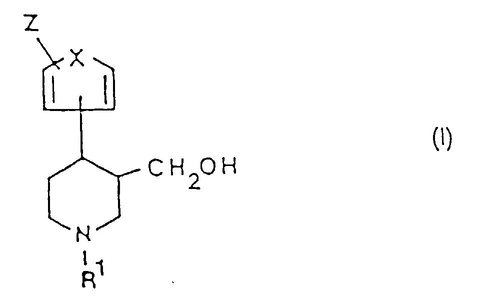 worin X die Bedeutung O, S oder NR hat, worin R Wasserstoff oder C₁₋₄-Alkyl bedeutet; Z Wasserstoff oder geradkettiges oder verzweigtes C₁₋₈-Alkyl bedeutet; und R¹ Wasserstoff oder geradkettiges oder verzweigtes C₁₋₈-Alkyl bedeutet.

2. Verbindung der Formel II 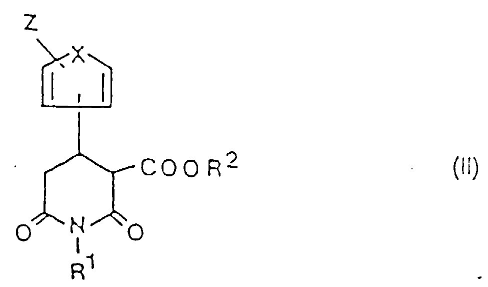 worin X die Bedeutung O, S, oder NR hat, worin R Wasserstoff oder C₁₋₄-Alkyl bedeutet; Z Wasserstoff oder geradkettiges oder verzweigtes C₁₋₈-Alkyl bedeutet; R¹ Wasserstoff oder geradkettiges oder verzweigtes C₁₋₈-Alkyl bedeutet; und R² C₁₋₂-Alkyl bedeutet.

3. Verfahren zur Herstellung einer Verbindung nach Anspruch 1, umfassend die Umsetzung einer Verbindung der Formel IV
R³OCCH₂COOR² IV
mit einer Verbindung der Formel V 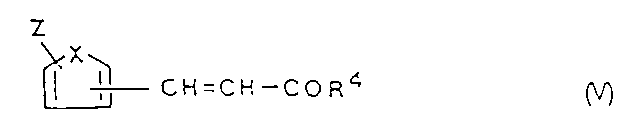 unter basischen Bedingungen, worin X, Z, R, und R² die vorstehend definierten Bedeutungen haben und R³ C₁₋₂-Alkoxy bedeutet, wenn R⁴ die Bedeutung NHR¹ hat, oder R³ NHR¹ bedeutet, wenn R⁴ die Bedeutung C₁₋₂-Alkoxy hat, wobei R¹ die vorstehend definierte Bedeutung hat, unter Bildung einer Verbindung der Formel II 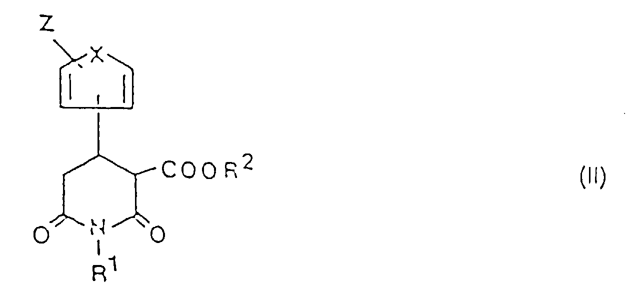 worin X, Z, R, R¹ und R² die vorstehend definierten Bedeutungen haben, sowie die Reduktion einer Verbindung der Formel II mit einem Metallhydrid, wie LiAlH₄, unter Bildung einer Verbindung der Formel I.

4. Verfahren zur Herstellung einer Verbindung nach Anspruch 1, umfassend die Umsetzung einer Verbindung der Formel VII 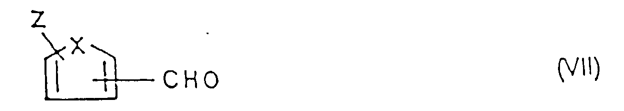 mit einer Verbindung der Formel VIII
R²OOCCH₂CONHR¹ VIII
in Essigsäureethylester unter Verwendung von Ethoxid oder Methoxid als Base, wobei X, Z, R, R¹ und R² die vorstehend definierten Bedeutungen haben, unter Bildung einer Verbindung der Formel II 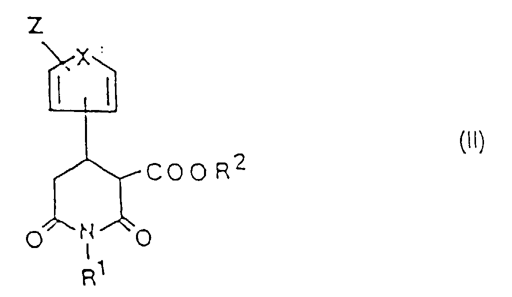 in der X, Z, R, R¹ und R² die in Anspruch 2 definierten Bedeutungen haben, und die Reduktion einer Verbindung der Formel II mit einem Metallhydrid, wie LiAlH₄ unter Bildung einer Verbindung der Formel I.

5. Verfahren zur Herstellung von 3-Hydroxymethyl-1-methyl-4-(2-thienyl)-piperidin, umfassend die Umsetzung von Arecolin und HBr mit 2-Bromthiophen in Gegenwart von Mg-Spänen unter Bildung der Verbindung 3-Methoxycarbonyl-1-methyl-4-(2-thienyl)-piperidin und Reduktion dieser Verbindung mit LiAlH₄.

6. Verbindung der Formel II, wobei es sich um 3-Ethoxycarbonyl-1-pentyl-4-(2-thienyl)-piperidin-2,6-dion handelt.

7. Verbindung der Formel I, wobei es sich um 3-Hydroxymethyl-1-methyl-4-(2-thienyl)-piperidin handelt.

8. Verbindung der Formel I, wobei es sich um 3-Hydroxymethyl-1-pentyl-4-(3-thienyl)-piperidin handelt.

9. Verbindung der Formel I, wobei es sich um 3-Ethoxycarbonyl-1-pentyl-4-(3-thienyl)-piperidin-2,6-dion handelt.

10. Verbindung der Formel I, wobei es sich um 3-Hydroxymethyl-1-pentyl-4-(2-thienyl)-piperidin handelt.

11. Verbindung der Formel I, wobei es sich um 1-Butyl-3-hydroxymethyl-4-(2-thienyl)-piperidin handelt.

12. Verbindung der Formel I, wobei es sich um 1-Butyl-3-hydroxymethyl-4-(5-methyl-2-furanyl)-piperidin handelt.

13. Verbindung der Formel I, wobei es sich um 1-Butyl-3-hydroxymethyl-4-(3-thienyl)-piperidin handelt.

14. Verbindung der Formel I, wobei es sich um 3-Hydroxymethyl-1-(2-methyl-butyl)-4-(3-thienyl)-piperidin handelt.

15. Verbindung der Formel I, wobei es sich um 1-Butyl-3-hydroxymethyl-4-(1-methyl-2-pyrrolyl)-piperidin handelt.

## Revendications

1. Un composé de formule I 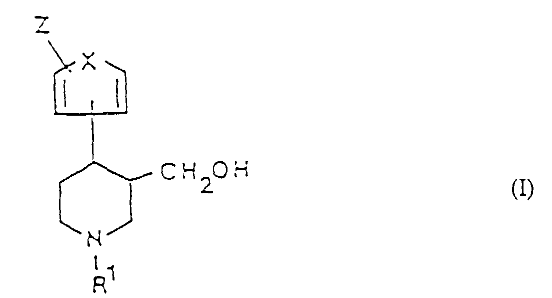 où X est O, S ou NR, où R est l'hydrogène ou un C₁₋₄-alkyle ; et Z est l'hydrogène ou un C₁₋₈-alkyle linéaire ou ramifié ; et R¹ est l'hydrogène ou un C₁₋₈-alkyle linéaire ou ramifié.

2. Un composé de formule II 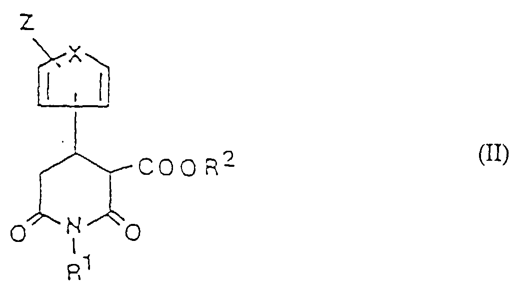 où X est O, S ou NR, où R est l'hydrogène ou un C₁₋₄-alkyle ; et Z est l'hydrogène ou un C₁₋₈-alkyle linéaire ou ramifié ; et R¹ est l'hydrogène ou un C₁₋₈-alkyle linéaire ou ramifié ; et R² est un C₁₋₂-alkyle.

3. Un processus pour la préparation d'un composé selon la revendication 1, comprenant la mise en réaction d'un composé de formule IV
R³OCCH₂COOR² (IV)
avec un composé de formule V 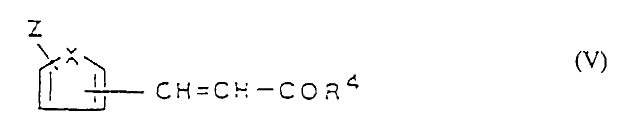 dans des conditions basiques, où X, Z, R et R² ont les sens donnés plus haut et R³ étant un C₁₋₂-alcoxy lorsque R⁴ est NHR¹ ou R³ étant NHR' lorsque R⁴ est un C₁₋₂-alcoxy, où R¹ a le sens donné plus haut, pour former un composé de formule II 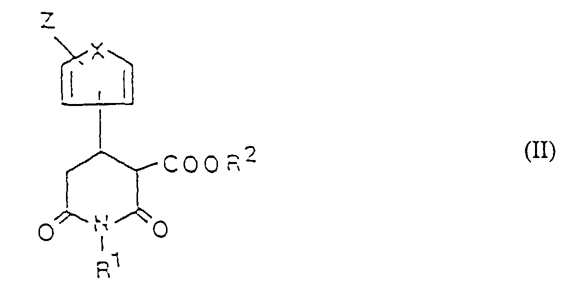 où X, Z, R, R¹ et R² ont les sens donnés plus haut, et la réduction d'un composé de formule II avec un hydrure métallique, par exemple LiAlH₄, pour former un composé de formule I.

4. Un processus pour la préparation d'un composé selon la revendication 1, comprenant la mise en réaction d'un composé de formule VII 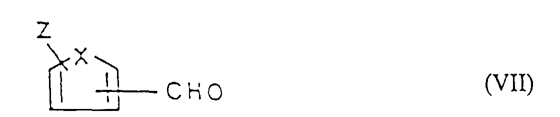 avec un composé de formule VIII
R²OOCCH₂CONHR¹ (VIII)
dans l'acétate d'éthyle en utilisant l'éthoxyde ou le méthoxyde comme base, où X, Z, R, R¹ et R² ont les sens donnés plus haut, pour former un composé de formule II 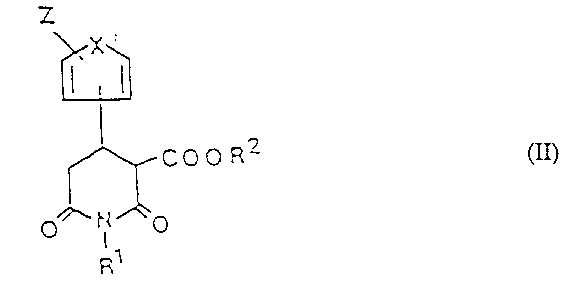 où X, Z, R, R¹ et R² ont les sens donnés à la revendication 2, et la réduction d'un composé de formule II avec un hydrure métallique, par exemple LiAlH₄, pour former un composé de formule I.

5. Un processus pour la préparation de la 3-hydroxyméthyl-1-méthyl-4-(2-thiényl)-pipéridine, comprenant la mise en réaction d'arécoline et de HBr avec du 2-bromothiophène en présence de copeaux de Mg, pour former le composé 3-méthoxycarbonyl-1-méthyl-4-(2-thiényl)-pipéridine, et la réduction de ce composé avec LiAlH₄.

6. Un composé de formule II, qui est la 3-éthoxycarbonyl-1-pentyl-4-(2-thiényl)-pipéridine-2,6-dione.

7. Un composé de formule I, qui est la 3-hydroxyméthyl-1-méthyl-4-(2-thiényl)-pipéridine.

8. Un composé de formule I, qui est la 3-hydroxyméthyl-1-pentyl-4-(3-thiényl)-pipéridine.

9. Un composé de formule II, qui est la 3-éthoxycarbonyl-1-pentyl-4-(3-thiényl)-pipéridine-2,6-dione.

10. Un composé de formule I, qui est la 3-hydroxyméthyl-1-pentyl-4-(2-thiényl)-pipéridine.

11. Un composé de formule I, qui est la 1-butyl-3-hydroxyméthyl-4-(2-thiényl)-pipéridine.

12. Un composé de formule I, qui est la 1-butyl-3-hydroxyméthyl-4-(5-méthyl-2-furanyl)-pipéridine.

13. Un composé de formule I, qui est la 1-butyl-3-hydroxyméthyl-4-(3-thiényl)-pipéridine.

14. Un composé de formule I, qui est la 3-hydroxyméthyl-1-(2-méthyl-butyl)-4-(3-thiényl)-pipéridine.

15. Un composé de formule I, qui est la 1-butyl-3-hydroxyméthyl-4-(1-méthyl-2-pyrrolyl)-pipéridine.
